# EUROPEAN PATENT APPLICATION

(11) **EP 1 925 334 A1**
(43) Date of publication of application: **28.05.2008**
(21) Application number: 06797924.5
(22) Date of filing: 13.09.2006
(51) Int. Cl.: A61N 1/30

(54) **IONTOPHORESIS DEVICE AND POWER SUPPLY DEVICE FOR IONTOPHORESIS DEVICE**

(30) Priority: 14.09.2005 JP 2005267588
(71) Applicant: TTI ELLEBEAU, INC., Tokyo 140-0002 (JP)
(72) Inventor: NAKAYAMA, Mizuo c/o TTI ellebeau, Inc., Shinagawa-ku, Tokyo 140-0002 (JP); MATSUMURA, Takehiko, c/o TTI ellebeau, Inc., Shinagawa-ku, Tokyo 140-0002 (JP); AKIYAMA, Hidero c/o TTI ellebeau, Inc., Shinagawa-ku, Tokyo 140-0002 (JP); MATSUMURA, Akihiko c/o TTI ellebeau, Inc., Shinagawa-ku, Tokyo 140-0002 (JP)
(74) Representative: Geyer, Ulrich F.
(86) International application number: PCT/JP2006/318174
(87) International publication number: WO 2007/032398

(57) **Abstract**

An iontophoresis device includes: an electric power source device; a drug administrating means including a working electrode assembly holding an ionic drug, the working electrode assemblybeing connected to the electric power source device, and a non-working electrode assembly holding an ionic drug, the non-working electrode assembly serving as a counter electrode of the working electrode assembly; and a current control unit for controlling a current flowing in each of the electrode assemblies by means of a program in accordance with a preset pattern. The ionic drug is released from each of the electrode assemblies in accordance with a current flowing in each of the electrode assemblies to be transdermally administered to an organism. Thereby, the drug can be administered in accordance with the living pattern of a patient and the circadian rhythm of the drug.

## Description

### [Technical Field]

The present invention relates to an iontophoresis device for administering a drug ion to an organism and an electric power source device for an iontophoresis device.

### [Background Art]

Iontophoresis is one method of permeating a drug into an organism from the skin or mucosa of the organism (see JP 63-35266 A, JP 04-297277 A, JP 2000-229128 A, JP 2000-229129 A, JP 2000-237327 A, JP 2000-237328 A, WO 03/0374254 A1). The iontophoresis includes a working electrode assembly having a drug solution holding portion holding a drug solution and a non-working electrode assembly as a counter electrode of the working electrode assembly. A voltage having the same conductivity type as that of a drug ion in the drug solution holding portion is applied to the working electrode assembly in a state where the drug solution is brought into close contact with the skin or mucosa to electrically drive the drug ion so that the drug ion is transferred into an organism via the skin or mucosa of the organism.

A patch-type structure is one possible structure for each of the working electrode assembly and the non-working electrode assembly.

The term "patch" conventionally refers to a cloth-like section having adhesiveness on one surface mainly containing a substance such as a drug or an antigen. The structure of the patch may have some degree of thickness, or the patch may have no adhesiveness.

As described in, for example, JP 2002-532540 A, a nicotine patch containing nicotine as an antismoking auxiliary agent has been known as an example of such patch. In addition, as described in JP 2005-510488 A, some transdermal absorption patches are used as local anesthetic drugs using morphine hydrochloride and the like.

Furthermore, a drug tape has been also developed, in which a film is constituted in such a manner that the amount of a drug to be released reaches its peak, for example, 6 hours after the sticking of the tape.

However, conventionally, the amount of a drug to be administered cannot be finely changed. For example, a drug cannot be administered in accordance with the living pattern of a patient, an individual difference, an onset time (for example, dawn in the case of asthmatic attack), a circadian rhythm, or the like. In particular, an oral medicine may apply an excessive load to a stomach. In addition, when one attempts to manage the amount of a drug to be administered by peeling a patch-type iontophoresis device off a skin a predetermined time period after the device has been stuck to the skin, the amount of the drug to be administered may exceed an allowable value in the case where the device is erroneously stuck for a long time period owing to, for example, the fact that he or she forgets to peel the device off the skin.

### [Disclosure of the Invention]

The present invention has been made with a view to solving the conventional problems, and an object of the present invention is to provide an iontophoresis device capable of controlling the amount of a drug to be administered and the time at which the drug is administered in consideration of the living pattern of a patient and the circadian rhythm of the drug and an electric power source device for the iontophoresis device.

The present invention has solved the above problems, and according to one aspect of the present invention, there is provided an iontophoresis device including: an electric power source device; drug administering means including a working electrode assembly holding an ionic drug and a non-working electrode assembly holding an ionic drug as a counter electrode of the working electrode assembly, the drug administering means being connected to the electric power source device; and current control means for program-controlling a current which flows to each of the electrode assemblies according to a preset pattern; characterized in that the ionic drug is released from each of the electrode assemblies according to a current which flows to each of the electrode assemblies to thereby transdermally administer the ionic drug into an organism.

According to another aspect of the present invention, there is provided an iontophoresis device characterized in that an output pattern of the current control means is rewritable from the outside without contact.

According to still another aspect of the present invention, there is provided an iontophoresis device characterized in that the working electrode assembly and the non-working electrode assembly each are of a patch type.

According to yet another aspect of the present invention, there is provided an iontophoresis device characterized in that the working electrode assembly includes: a working electrode connected to the electric power source with the same polarity as that of a charged ion of the ionic drug; an electrolyte solution holding portion holding an electrolyte solution, the electrolyte solution holding portion being placed on a front surface of the working electrode; a second ion exchange membrane selecting an ion having a polarity opposite to that of the charged ion of the ionic drug, the second ion exchange membrane being placed on a front surface of the electrolyte solution holding portion; a drug solution holding portion holding the ionic drug, the drug solution holding portion being placed on a front surface of the second ion exchange membrane; and a first ion exchange membrane selecting an ion having the same polarity as that of the charged ion of the ionic drug, the first ion exchange membrane being placed on a front surface of the drug solution holding portion, and the non-working electrode assembly includes: the non-working electrode connected to the electric power source having a polarity opposite to that of the charged ion of the ionic drug; a second electrolyte solution holding portion holding a second electrolyte solution, the second electrolyte solution holding portion being placed on a front surface of the non-working electrode; a third ion exchange membrane selecting an ion having the same polarity as that of the charged ion of the ionic drug, the third ion exchange membrane being placed on a front surface of the second electrolyte solution holding portion; a third electrolyte solution holding portion holding a third electrolyte solution, the third electrolyte solution holding portion being placed on a front surface of the third ion exchange membrane; and a fourth ion exchange membrane selecting an ion having a polarity opposite to that of the charged ion of the ionic drug, the fourth ion exchange membrane being placed on a front surface of the third electrolyte solution holding portion.

According to still yet another aspect of the present invention, there is provided an electric power source device for an iontophoresis device including a working electrode assembly and a non-working electrode assembly each used for administering an ionic drug by iontophoresis, the electric power source device being characterized by including: a battery; and a current control circuit for program-controlling a current from the battery to each of the electrode assemblies according to a preset pattern.

According to still yet another aspect of the present invention, there is provided an antenna for making an output pattern of the current control circuit rewritable from the outside without contact.

According to still yet another aspect of the present invention, there is provided an electric power source device for an iontophoresis device characterized in that the current control circuit is disposed radially outside the battery and side by side next to the battery.

According to further another aspect of the present invention, there is provided an electric power source device for an iontophoresis device characterized in that the battery is disposed off a center of the working electrode assembly in a plan view and the current control circuit is disposed next to the battery on a side opposite to a direction of a shift of the battery from the center of the working electrode assembly.

According to yet further another aspect of the present invention, there is provided an electric power source device for an iontophoresis device characterized in that the current control circuit is disposed over the battery in a thickness direction of the battery.

According to the present invention, a current amount and a discharge duration are controlled by means of a program in conformance with a pattern determined by a doctor by utilizing the fact that the amount of a drug to be administered by an iontophoresis device is proportional to a current amount, so the amount of a drug to be administered and the time at which the drug is administered are controlled. As a result, the drug can be administered in accordance with, for example, the living pattern of a patient and a circadian rhythm.

Furthermore, even when an iontophoresis device is erroneously stuck to a skin for a long time period, the amount of a drug to be administered can be limited to an allowable value or less by causing a current amount to be zero at a predetermined discharge duration (administration time).

### [Brief Description of the Drawings]

[Fig. 1] A plan view showing an iontophoresis device according to a first embodiment of the present invention.
[Fig. 2] An enlarged sectional view taken along the line II-II of Fig. 1.
[Fig. 3] An enlarged sectional view taken along the line III-III of Fig. 1.
[Fig. 4] A circuit diagram showing an electrical system in the iontophoresis device.
[Fig. 5] A circuit diagram showing a current control circuit in the iontophoresis device.
[Fig. 6] A view showing a current pattern in the iontophoresis device.
[Fig. 7] A plan view showing a DC electric power source according to a second embodiment.
[Fig. 8] A sectional view similar to Fig. 2 showing a DC electric power source according to a third embodiment.

### [Best Mode for carrying out the Invention]

Hereinafter, the best mode for carrying out the present invention will be described in detail with reference to the drawings.

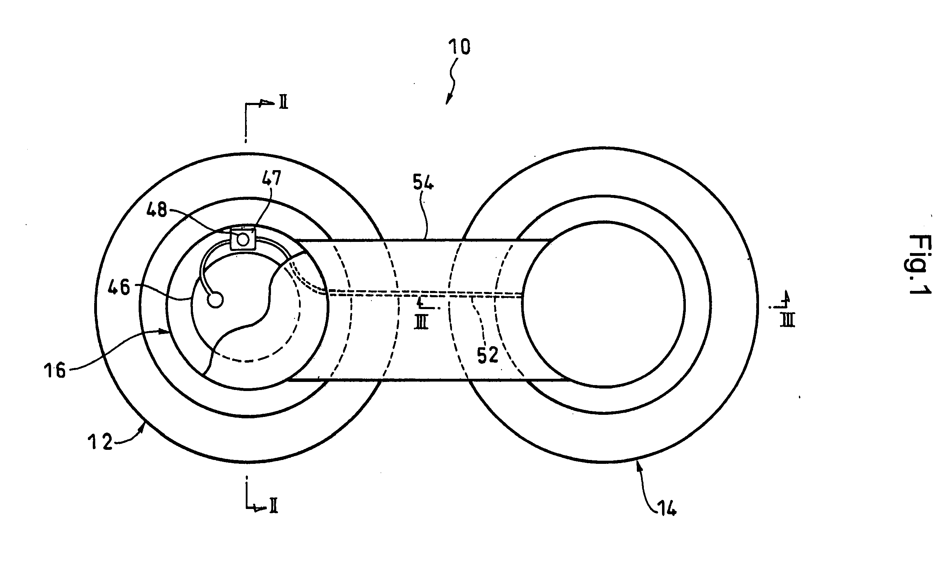

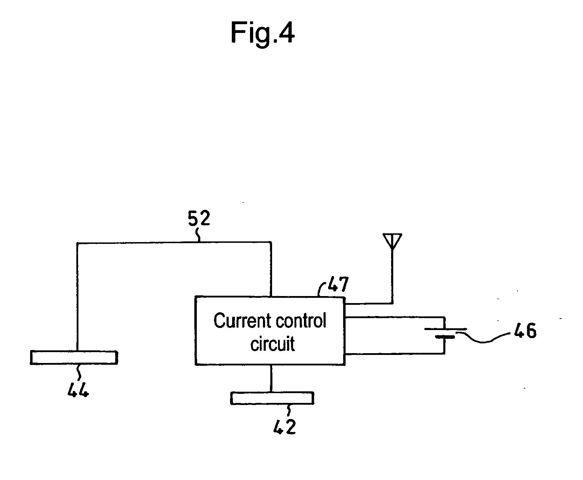

As shown in Figs. 1 and 4, an iontophoresis device 10 according to the best mode is constituted by a working electrode assembly 12 and a non-working electrode assembly 14 each used for administering an ionic drug, and a DC electric power source 16 connected to the electrode assemblies 12 and 14 with opposite polarities.

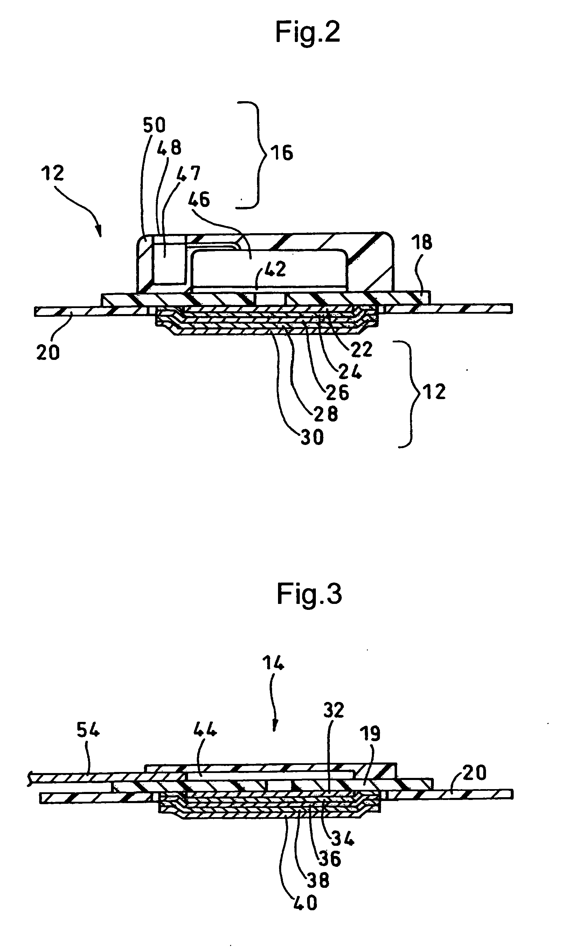

Fig. 2 shows the section of the working electrode assembly 12. The working electrode assembly 12 is constituted by laminating a working electrode 22, an electrolyte solution holding portion 24, a second ion exchange membrane 26, a drug solution holding portion 28, and a first ion exchange membrane 30 in the stated order on the lower side in Fig. 2 of a base sheet 18, and its plane shape is a circle of 10 to 40 mm in diameter. A circular adhesive sheet 20 is arranged on the lower surface in Fig. 2 of the base sheet 18 so as to surround the working electrode 22 to the first ion exchange membrane 30.

The base sheet 18 is preferably constituted by a resin material which is hard, insulative, and elastic such as a polyethylene terephthalate (PET) resin, and is adapted to press the working electrode 22 to the first ion exchange membrane 30 against the skin or mucosa of an organism when the sheet 18 is pulled with the adhesive sheet 20.

The working electrode 22 is desirably constituted by a conductive paint applied to the one surface of the base sheet 18 and blended with a nonmetal conductive filler such as a carbon paste. The working electrode 22 can be constituted by a copper plate or a metal thin film, but a metal eluted from the plate or the thin film may transfer to an organism upon administration of a drug. Therefore, the working electrode 22 is preferably nonmetallic.

The electrolyte solution holding portion 24 is constituted by, for example, an electrolytic paint applied to the working electrode 22. The electrolytic paint is a paint containing an electrolyte, and medical agents such as ascorbic acid (vitamin C) and sodium ascorbate, and organic acids such as lactic acid, oxalic acid, malic acid, succinic acid, and fumaric acid and/or salts thereof are particularly preferably used for the electrolyte. The use of such electrolyte can suppress the generation of an oxygen gas or a hydrogen gas. In addition, blendingmultiple kinds of electrolytes serving as a combination of buffer electrolyte solutions upon dissolution into a solvent can suppress a change in pH during energization.

The electrolytic paint is blended with a hydrophilic polymer such as polyvinyl alcohol, polyacrylic acid, polyacrylamide, or polyethylene glycol in order to improve the application property and film-forming property of the paint, and is blended with an appropriate amount of solvent such as water, ethanol, or propanol for adjusting the viscosity of the electrolytic paint. The paint may be blended with an appropriate additional component such as a thickener, a thixotropic agent, a defoaming agent, a pigment, a flavor, or a coloring agent.

The second ion exchange membrane 26 is formed by applying a second ion exchange paint to the electrolyte solution holding portion 24.

The second ion exchange paint is a paint containing an ion exchange resin into which an ion exchange group using, as a counter ion, an ion having a conductivity type opposite to that of a drug ion in the drug solution holding portion 28 to be described later is introduced. In the case where a drug whose drug component dissociates to plus drug ions is used in the drug solution holding portion 28, the paint is blended with an anion exchange resin. On the other hand, in the case where a drug whose drug component dissociates to minus drug ions is used, the paint is blended with a cation exchange resin.

The drug solution holding portion 28 is composed of a drug paint applied to the second ion exchange membrane 26. The drug paint is a paint containing a drug (including a precursor for the drug) whose drug component dissociates to plus or minus ions (drug ions) as a result of, for example, dissolution into a solvent such as water. Examples of a drug whose drug component dissociates to plus ions can include lidocaine hydrochloride as an anesthetic drug and morphine hydrochloride as an anesthetic drug. Examples of a drug whose drug component dissociates to minus ions can include ascorbic acid as a vitamin agent.

The first ion exchange membrane 30 is formed of a first ion exchange paint applied to the drug solution holding portion 28. The first ion exchange paint is a paint containing an ion exchange resin into which an ion exchange group using, as a counter ion, an ion having the same conductivity type as that of the drug ion in the drug solution holding portion 28 is introduced. In the case where a drug whose drug component dissociates to plus/minus drug ions is used in the drug solution holding portion 28, the paint is blended with a anion/cation exchange resin.

An ion exchange resin obtained by introducing a cation exchange group (an exchange group using a cation as a counter ion) such as a sulfonic group, a carboxylic group, or a phosphoric group into a polymer having a three-dimensional network structure such as a hydrocarbon-based resin (for example, a polystyrene resin or an acrylic resin) or a fluorine-based resin having a perfluorocarbon skeleton can be used as the cation exchange resin without any limitation.

An ion exchange resin obtained by introducing an anion exchange group (an exchange group using an anion as a counter ion) such as a primary amino group, a secondary amino group, a tertiary amino group, a quaternary ammonium group, a pyridyl group, an imidazole group, a quaternary pyridinium group, or a quaternary imidazolium group into a polymer having a three-dimensional network structure similar to that in the case of the cation exchange resin can be used as the anion exchange resin without any limitation.

Fig. 3 is an enlarged view showing that the non-working electrode assembly 14 is constituted by laminating a non-working electrode 32, a second electrolyte solution holding portion 34, a third ion exchange membrane 36, a third electrolyte solution holding portion 38, and a fourth ion exchange membrane 40 arranged on the lower side in the figure of a non-working base sheet 19 similar to the base sheet 18 in the stated order, and its plane shape is the same as that of the working electrode assembly 12.

The non-working electrode 32 has the same constitution as that of the working electrode 22 in the working electrode assembly 12, and the constitutions and components of the second electrolyte solution holding portion 34 and the third electrolyte solution holding portion 38 are the same as those of the electrolyte solution holding portion 24.

Furthermore, the third ion exchange membrane 36 is formed of an ion exchange paint applied to the second electrolyte solution holding portion 34. The ion exchange paint is the same as the first ion exchange paint of which the first ion exchange membrane 30 is formed, and the third ion exchange membrane 36 functions as an ion exchange membrane similar to the first ion exchange membrane 30.

The fourth ion exchange membrane 40 is formed of the same second ion exchange paint as that described above applied to the third electrolyte solution holding portion 38. The fourth ion exchange membrane 40 functions as an ion exchange membrane similar to the second ion exchange membrane 26.

Aworking electrode terminal 42 is arranged on the upper surface in Fig. 2 of the base sheet 18 in the working electrode assembly 12, and conduction is established between the working electrode terminal 42 and the working electrode 22 of the working electrode assembly 12 through a through-hole arranged on the base sheet 18.

Similarly, a non-working electrode terminal 44 is arranged on the upper surface in Fig. 3 of the non-working base sheet 19 in the non-working electrode assembly 14, and conduction is established between the non-working electrode terminal 44 and the non-working electrode 32 of the non-working electrode assembly 14 through a through-hole formed on the non-working base sheet 19.

The DC electric power source 16 is arranged to cover the upper side of the working electrode terminal 42.

The DC electricpower source 16 is constituted by: a coinbattery 46; a current control circuit 47 including a programmable microprocessor or programmable controller constituted by, for example, a 1 chip IC in which the output pattern of a current is rewritable with radio from the outside and a minute antenna 48 of, for example, a loop shape; and a mold resin 50 composed of an insulating material for integrally molding them.

The coin battery 46 is arranged at substantially the center of the circular working electrode assembly 12 in a plan view, and the current control circuit 47 is placed outside the coin battery 4 6 and laterally next to the coin battery 46. Each of the coin battery 46 and the current control circuit 47 is entirely molded into a flat circular shape having an outside diameter substantially equal to that of the circular working electrode assembly 12 by the mold resin 50.

In the DC electric power source 16, as shown also in Fig. 4 (a circuit diagram), the coin battery 46 is connected to the current control circuit 47, and the cathode side of the circuit is connected to the working electrode terminal 42 and the anode side of the circuit is connected to the non-working electrode terminal 44 on the side of the non-working electrode assembly 14 through a lead wire 52.

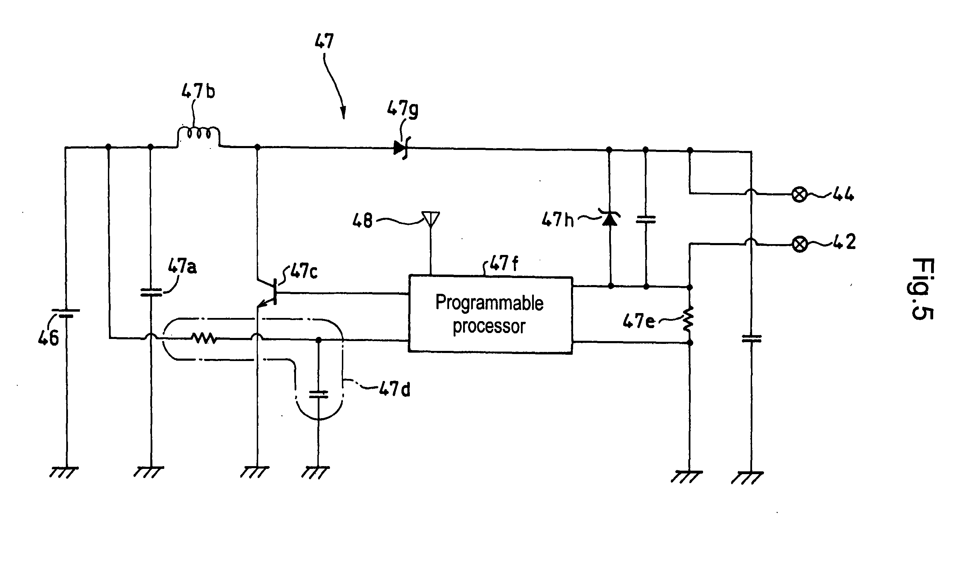

As shown in detail in Fig. 5, the current control circuit 47 mainly includes: a capacitor 47a for accumulating the charge of the battery 46; a coil 47b for collectively discharging the charge accumulated in the capacitor 47a; a switching transistor 47c for turning the output side of the coil 47b on or off; an RC filter 47d; a feedback resistor 47e for detecting a current flowing between the electrode terminals 42 and 44 (that is, in a patient); and a programmable processor 47f working as a boosting converter for turning the switching transistor 47c on or off in such a manner that the voltage across the feedback resistor 47e becomes a set value. In the figure, reference symbol 47g denotes a reverse flow preventing diode and reference symbol 47h denotes a protecting diode.

Reference numeral 54 in Fig. 1 denotes a coupling belt composed of, for example, a PET film for coupling the base sheet 18 and the non-working base sheet 19. The lead wire 52 is arranged to pass through the inside of the coupling belt 54 or to be along the surface of the belt.

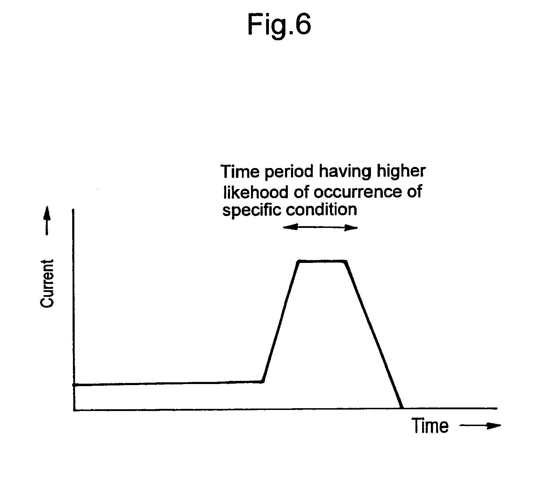

Fig. 6 shows an example of a current pattern preset in the current control circuit 47, that is, an administration pattern. Thus, a drug can be administered in accordance with an attack time at dawn even when a patch is stuck before sleeping at night. Furthermore, even when one forgets to peel the patch off the skin, the amount of the drug to be administered cannot be excessive. It should be noted that a current pattern is rewritable for each patch with radio from the outside through the antenna 48. In addition, a pattern can be fixed for each patch while the antenna 48 is omitted.

In this embodiment, the DC electric power source 16 includes the coin battery 46 and the electric power source circuit 47 each molded into a flat shape by the mold resin 50 and each having a diameter substantially equal to that of the working electrode assembly 12 in a plan view. As a result, the patch-type working electrode assembly 12 can be constituted without any increase in entire size of the assembly.

In the above embodiment, the DC electric power source 16 is arranged on the side of the working electrode assembly 12. However, the present invention is not limited thereto. The DC electric power source 16 may be arranged on the side of the non-working electrode assembly 14, or may be arranged on each of the working electrode assembly 12 and the non-working electrode assembly 14.

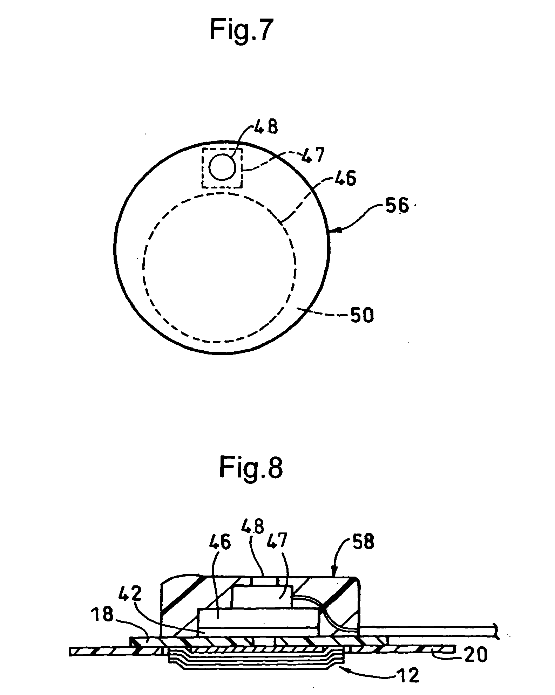

In the DC electric power source 16, the coin battery 46 is arranged at the center of the working electrode assembly 12 in a plan view, and the electric power source circuit 47 is placed outside and next to the coin battery 46. However, the present invention is not limited thereto. For example, like a DC electric power source 56 in a second embodiment shown in Fig. 7, the coin battery 46 may be shifted from the center of the circular working electrode assembly 12 in a plan view to one side and the electric power source circuit 47 may be arranged on the opposite side. In this case, the outer diameter of the mold resin 50 can be reduced.

Furthermore, like a DC electric power source 58 in a third embodiment shown in Fig. 8, the electric power source circuit 47 may be arranged and molded on the upper side in Fig. 6 of the coin battery 46. This case is applicable to the case where the diameter of the working electrode assembly 12 in a plan view is small.

In each of the above embodiments, the present invention is applied to an iontophoresis device constituted by a patch-type working electrode assembly and a non-working electrode assembly. However, the object to which the present invention is applicable is not limited thereto. A battery is not limited to a coin battery. In addition, the antenna 48 can be omitted by making the output pattern of the circuit 47 unrewritable from the outside after the current control circuit 47 has been incorporated.

### [Industrial Applicability]

Drug ion can be administered to an organism in accordance with the living pattern of a patient and the circadian rhythm of the drug.

## Claims

1. An iontophoresis device comprising:
an electric power source device;
drug administering means comprising a working electrode assembly holding an ionic drug and a non-working electrode assembly holding an ionic drug as a counter electrode of the working electrode assembly, the drug administering means being connected to the electric power source device; and
current control means for program-controlling a current which flows to each of the electrode assemblies according to a preset pattern;
whereby the ionic drug is released from each of the electrode assemblies according to a current which flows to each of the electrode assemblies to thereby transdermally administer the ionic drug into an organism.

2. The iontophoresis device according to claim 1, **characterized in that** an output pattern of the current control means is rewritable from the outside without contact.

3. The iontophoresis device according to claim 1 or 2, **characterized in that** the working electrode assembly and the non-working electrode assembly each are of a patch type.

4. The iontophoresis device according to any one of claims 1 to 3, **characterized in that**
the working electrode assembly comprises:
a working electrode connected to the electric power source with the same polarity as that of a charged ion of the ionic drug;
an electrolyte solution holding portion holding an electrolyte solution, the electrolyte solution holding portion being placed on a front surface of the working electrode;
a second ion exchange membrane selecting an ion having a polarity opposite to that of the charged ion of the ionic drug, the second ion exchange membrane being placed on a front surface of the electrolyte solution holding portion;
a drug solution holding portion holding the ionic drug, the drug solution holding portion being placed on a front surface of the second ion exchange membrane; and
a first ion exchange membrane selecting an ion having the same polarity as that of the charged ion of the ionic drug, the first ion exchange membrane being placed on a front surface of the drug solution holding portion, and
the non-working electrode assembly comprises:
the non-working electrode connected to the electric power source having a polarity opposite to that of the charged ion of the ionic drug;
a second electrolyte solution holding portion holding a second electrolyte solution, the second electrolyte solution holding portion being placed on a front surface of the non-working electrode;
a third ion exchange membrane selecting an ion having the same polarity as that of the charged ion of the ionic drug, the third ion exchange membrane being placed on a front surface of the second electrolyte solution holding portion;
a third electrolyte solution holding portion holding a third electrolyte solution, the third electrolyte solution holding portion being placed on a front surface of the third ion exchange membrane; and
a fourth ion exchange membrane selecting an ion having a polarity opposite to that of the charged ion of the ionic drug, the fourth ion exchange membrane being placed on a front surface of the third electrolyte solution holding portion.

5. An electric power source device for an iontophoresis device comprising a working electrode assembly and a non-working electrode assembly each used for administering an ionic drug by iontophoresis, the electric power source device being **characterized by** comprising:
a battery;
a current control circuit for program-controlling a current from the battery to each of the electrode assemblies according to a preset pattern.

6. The electric power source device for an iontophoresis device according to claim 5 further comprising an antenna for making an output pattern of the current control circuit rewritable from the outside without contact.

7. The electric power source device for an iontophoresis device according to claim 5 or 6, **characterized in that** the current control circuit is disposed radially outside the battery and side by side next to the battery.

8. The electric power source device for an iontophoresis device according to claim 5 or 6, **characterized in that** the battery is disposed off a center of the working electrode assembly in a plan view and the current control circuit is disposed next to the battery on a side opposite to a direction of a shift of the battery from the center of the working electrode assembly.

9. The electric power source device for an iontophoresis device according to claim 4, **characterized in that** the current control circuit is disposed over the battery in a thickness direction of the battery.

10. The electric power source device for an iontophoresis device according to any of claims 5 to 9, **characterized in that** at least the battery and the current control circuit is formed into flat by mold resin with outer size as a whole being almost same as the working electrode assembly.
